# EUROPEAN PATENT APPLICATION

(11) **EP 4 438 035 A1**
(43) Date of publication of application: **02.10.2024**
(21) Application number: 22899157.6
(22) Date of filing: 19.10.2022
(51) Int. Cl.: A61K 9/08, A61K 47/10, A61K 47/26, A61K 38/00, A61K 38/27, A61K 47/64

(54) **HIGH CONCENTRATION ADMINISTRATION FORMULATION OF HGH FUSION PROTEIN**

(30) Priority: 26.11.2021 KR 20210165734
(71) Applicant: Genexine, Inc., Seoul 07789 (KR); Handok Inc., Seoul 06235 (KR)
(72) Inventor: PARK, Chanwoong, Siheung-si, Gyeonggi-do 15010 (KR); KIM, Ki-Yong, Yongin-si, Gyeonggi-do 16938 (KR)
(74) Representative: Dehns
(86) International application number: PCT/KR2022/095143
(87) International publication number: WO 2023/096475

(57) **Abstract**

The present invention relates to a high-concentration administration formulation of hGH fusion protein containing poloxamer 188 and polysorbate 80. When containing poloxamer 188 and polysorbate 80 according to the present invention, a high-concentration formulation of hGH fusion protein having significantly reduced aggregation reactions and improved storage stability can be prepared.

## Description

### [Technical Field]

The present invention relates to a high-concentration human growth hormone (hGH) fusion protein formulation for administration, and more particularly, to a high-concentration hGH fusion protein formulation for administration having significantly reduced aggregation reactions even in a high-concentration hGH formulation to which an immunoglobulin Fc polypeptide (hereinafter referred interchangeably with "hyFc") is fused because the high-concentration formulation contains poloxamer 188 and polysorbate 80.

### [Background Art]

Growth hormone (also known as human growth hormone (hGH)) is a polypeptide consisting of 191 amino acids and is a hormone secreted by the anterior pituitary gland. Growth hormone participates in cell growth and regeneration by combining with a growth hormone receptor to express insulin-like growth factor-1 (IGF-1). Growth hormone is produced in the pituitary gland in a normal person's body, and its production is known to increase until puberty and gradually decrease with age.

Representative symptoms of growth hormone deficiency include adult growth hormone deficiency (AGHD) and pediatric growth hormone deficiency (PGHD). Adult growth hormone deficiency occurs idiopathically or when the patient's pituitary gland is damaged by radiation or surgery during the treatment of brain tumors, cerebral hemorrhage, and the like. When the growth hormone is not secreted properly, symptoms such as body weight loss, decreased bone mineral density, increased fat, decreased HDL, increased LDL, decreased muscle strength, and the like appear, thereby causing a decline in the quality of life. In adult patients with growth hormone deficiency, the concentration of IGF-1 in serum has a standard deviation score (SDS) of -2 or less (<_ -2 SDS) or falls within the 2.5 percentile (<_ 2.5 percentile) as compared to normal people in the same age group. The response value of the growth hormone in the blood may be measured by stimulation tests such as an insulin tolerance test (ITT), an arginine load (growth hormone-releasing hormone + arginine; GHRH + ARG) test, a glucagon test, an L-DOPA test, a clonidine test, and the like. When the peak growth hormone (GH) level is 11.0 µg/L or less in patients having a body mass index (BMI) of less than 25 kg/m², 8.0 µg/L or less in patients having a body mass index of 25 to 30 kg/m², or 4.0 µg/L or less in patients having a body mass index of more than 30 kg/m², the peak GH level is considered to be deficient (Guidelines for Use of Growth Hormone in Clinical Practice, Endocr. Pract. 2009; 15 (Suppl 2)). Pediatric growth hormone deficiency occurs when damage to the pituitary gland or developmental disorders are present in patients. When there is a growth hormone secretion disorder, short stature appears. In this case, the height is in the bottom 3% of the growth curve for individuals having the same age and gender, or a growth rate of 5 cm or less per year is shown, and symptoms such as hypoglycemia, reduced physical stamina, depression, mental immaturity, and the like appear. In the case where the height is lower than the mean by 3 standard deviations (SD) or more at the same age, where the height is lower than the average height of the parents by 1.5 SD or more, where the height is lower than the mean height by 2 SD or more and lower than the growth of individuals having the same age and gender by 1 SD or more for 1 year or more, where the height is lower than the growth of individuals having the same age and gender by 0.5 SD or more for 2 years or more, or where the short stature symptoms are not shown but the height is maintained at less than 2 SD for 1 year or more or 1.5 SD for 2 years or more, this case is determined to be pediatric growth hormone deficiency (Consensus guidelines for the diagnosis and treatment of GH deficiency in childhood and adolescence: summary statement of the GH Research Society. GH Research Society, J. Clin. Endocrinol. Metab., 2000 Nov; 85(11): 3990-3).

Growth hormone deficiency is mainly treated using growth hormones. When growth hormone treatment started for the first time in the 1950s, growth hormones were extracted from the pituitary gland of human cadavers. At this time, the supply was very limited and it was expensive because the amount of growth hormone extracted from one person was very small. With the development of gene recombinant technology, growth hormone synthesized in *Escherichia coli* was released (somatropin, 1981, Genentech, USA). The recombinant growth hormone drugs currently available in the USA include Genotropin by Pfizer, Humatrope by Eli Lilly, Nutropin by Genentech, Norditropin by Novo Nordisk, and the like.

Currently, growth hormone preparations are daily formulations, and especially for pediatric patients, it is inconvenient to inject the drug every day for a long treatment period of 3 to 4 years, and it is known that the mental stress caused by injection reduces the quality of life of patients. Also, the problem of patients unintentionally failing to receive injections frequently occurs and becomes the biggest factor hindering the effectiveness of treatment. In addition, it is known that the number of administration non-compliance increases significantly as the number of years of treatment increases (Endocrine Practice, 2008 Mar; 14(2): 143-54). Approximately two-thirds of patients fail to comply, resulting in lower compliance, and it is known that this actually reduces the height growth rate (PloS ONE, 2011 Jan; 6(1): e16223).

Due to these problems, there have been attempts to develop long-acting growth hormones using various techniques. Ascendis Pharma used the Transcon PEG platform, OPKO/Pfizer fused CTP to hGH, and Novo Nordisk fused albumin to increase the half-life of hGH. This technology made it possible to administer growth hormone injections once a week to patients who were receiving growth hormone injections daily.

In this regard, the present applicant has applied for and received a patent for the GX-H9 material, which has low complement-dependent and antibody-dependent toxicity and improved persistence of hGH by fusing hyFc, which is a combination of IgD and IgG4, to hGH (Registered US Patent No. 8,529,899). Subsequently, a patent was applied for a dosage regiment that allows GX-H9 to be administered once a week or once every two weeks (US Patent Publication No. 2021-0177945 and US Patent Publication No. 2019-0224281).

Meanwhile, in the early stages of clinical trials of GX-H9, it was formulated and supplied at a concentration of 30 mg/mL. Afterward, in the process of administering GX-H9 once every two weeks, the volume of GX-H9 administered increased. Therefore, the need for a high-concentration formulation has emerged.

Accordingly, the present inventors predicted the structural characteristics of GX-H9 through the sequence analysis of GX-H9, and based on the facts that hGH itself may form aggregates through hydrophobic interactions because it has many hydrophobic patches, and that proteins are adsorbed to gas/oil/solid surfaces in a drug product (DP) container to form a kind of protein film, and patches made by detachment of the proteins may form aggregates with insoluble particles, the present inventors made efforts to develop a formulation capable of preventing the formation of such aggregates. Therefore, the present invention was completed by confirming that the aggregate formation was significantly improved in a liquid formulation to which polysorbate 80 and poloxamer 188 are added together as surfactants.

### [Disclosure]

### [Technical Problem]

Therefore, it is one object of the present invention to provide a high-concentration formulation of hGH-hyFc fusion protein having significantly reduced protein aggregation reactions to maintain storage stability when preparing a high-concentration formulation of the hGH fusion protein.

### [Technical Solution]

To achieve the above object, according to an aspect of the present invention, there is provided a liquid pharmaceutical formulation including a human growth hormone (hGH) fusion protein, poloxamer 188, and polysorbate 80.

According to the present invention, the hGH fusion protein may be characterized by including hGH and an immunoglobulin Fc polypeptide.

According to the present invention, the hGH may be characterized by having an amino acid sequence of SEQ ID NO: 2.

According to the present invention, the immunoglobulin Fc polypeptide may be characterized by having an amino acid sequence of SEQ ID NO: 3.

According to the present invention, the hGH fusion protein may be characterized by having an amino acid sequence of SEQ ID NO: 1.

According to the present invention, the formulation may be characterized by including 30 mg/mL to 150 mg/mL of the hGH fusion protein.

According to the present invention, the formulation may be characterized by including 0.01% to 0.2% (w/v) poloxamer 188.

According to the present invention, the formulation may be characterized by including 0.01% to 0.2% (w/v) polysorbate 80.

According to the present invention, the formulation may be characterized by further including one or more excipients selected from the group consisting of histidine, arginine, glutamic acid, and sodium chloride (NaCl).

According to the present invention, the formulation may be characterized by further including a preservative.

According to the present invention, the preservative may be characterized by being at least one selected from the group consisting of m-cresol, phenol, and benzyl alcohol.

According to the present invention, the preservative may be characterized by being 1 mg/mL to 2 mg/mL of phenol.

According to the present invention, the formulation may be characterized by including 1 mM to 20 mM histidine.

According to the present invention, the formulation may be characterized by including 40 mM to 70 mM arginine.

According to the present invention, the formulation may be characterized by including 40 mM to 70 mM glutamic acid.

According to the present invention, the formulation may be characterized by including 80 mM to 100 mM sodium chloride.

According to the present invention, the formulation may be characterized by having a pH of 5.5 to 7.0.

According to the present invention, the formulation may be characterized by preferably having a pH of 6.0 to 6.9, more preferably a pH of 6.0 to 6.2.

According to the present invention, the formulation may be characterized by forming less than 1.5% of aggregates when stored in refrigerated conditions for 6 months.

According to the present invention, the formulation may be characterized by being administered subcutaneously or intramuscularly.

According to the present invention, the formulation may be characterized by being stored in a container selected from the group consisting of a bottle, a microtube, a bag, a vial, a cartridge, an injector, and a syringe.

### [Advantageous Effects]

When poloxamer 188 and polysorbate 80 according to the present invention are included, a high-concentration hGH fusion protein formulation having significantly reduced aggregation reactions and improved storage stability can be prepared.

### [Description of Drawings]

FIG. 1 shows the results of appearance analysis (visual inspection of insoluble foreign substances) to evaluate formulation stability according to the presence or absence of polysorbate 80 in various formulations of GX-H9.
FIG. 2 shows the results of analyzing fine particles (insoluble particulates) to evaluate formulation stability according to the presence or absence of polysorbate 80 in various formulations of GX-H9.
FIG. 3 shows the results of SE-UPLC analysis to evaluate formulation stability according to the presence or absence of polysorbate 80 in various formulations of GX-H9.
FIG. 4 shows the results of RP-HPLC analysis to evaluate formulation stability according to the presence or absence of polysorbate 80 in various formulations of GX-H9.
FIG. 5 shows the results of appearance analysis (visual inspection of insoluble foreign substances) to evaluate formulation stability according to the concentration of polysorbate 80 in the GX-H9 formulation.
FIG. 6 shows the results of analyzing fine particles to evaluate formulation stability according to the concentration of polysorbate 80 in the GX-H9 formulation.
FIG. 7 shows the results of purity (SE-UPLC, RP-HPLC) analysis to evaluate formulation stability according to the concentration of polysorbate 80 in the GX-H9 formulation.
FIG. 8 shows the results of appearance analysis (visual inspection of insoluble foreign substances) to evaluate formulation stability under various GX-H9 concentrations, polysorbate 80 concentrations, and preservative conditions.
FIG. 9A shows the results of purity (SE-UPLC, RP-HPLC) analysis to evaluate formulation stability under various GX-H9 concentration conditions.
FIG. 9B shows the results of purity (SE-UPLC, RP-HPLC) analysis to evaluate GX-H9 formulation stability under various polysorbate 80 concentration conditions.
FIG. 9C shows the results of purity (SE-UPLC, RP-HPLC) analysis to evaluate the stability of GX-H9 formulation according to various preservative treatments.

### [Best Mode]

Unless otherwise defined otherwise, all technical and scientific terms used in this specification have the same meaning as commonly understood by a person skilled in the art to which the present invention pertains. In general, the nomenclature used in this specification is well known and commonly used in the art.

In the present invention, it was confirmed that the formation of aggregates was significantly reduced when polysorbate 80 and poloxamer 188 were included as surfactants to prepare a high-concentration formulation of GX-H9.

Accordingly, according to one aspect, the present invention relates to a liquid pharmaceutical formulation including an hGH fusion protein, poloxamer 188, and polysorbate 80.

According to the present invention, the hGH fusion protein may be characterized by including hGH and an immunoglobulin Fc polypeptide.

As used in the present invention, the hGH fusion protein "GX-H9" refers to hGH-hyFc that is a human growth hormone fusion protein prepared by fusing hybrid Fc to human growth hormone (hGH). GX-H9, which is an hGH fusion protein, may be prepared according to the method disclosed in U.S. Patent No. 8,529,899.

According to the present invention, the hGH may be characterized by having an amino acid sequence of SEQ ID NO: 2, but the present invention is not limited thereto.

According to the present invention, the immunoglobulin Fc polypeptide may be characterized by having an amino acid sequence of SEQ ID NO: 3, but the present invention is not limited thereto.

According to the present invention, the hGH fusion protein may be characterized by having an amino acid sequence of SEQ ID NO: 1, but the present invention is not limited thereto.

According to the present invention, the formulation may be characterized by including 30 mg/mL to 150 mg/mL of the hGH fusion protein, and may be preferably characterized by including 60 mg/mL to 120 mg/mL of GX-H9, but the present invention is not limited thereto. For example, the formulation may include 30 mg/mL, 40 mg/mL, 50 mg/mL, 60 mg/mL, 70 mg/mL, 80 mg/mL, 90 mg/mL, 100 mg/mL, 110 mg/mL, 120 mg/mL, 130 mg/mL, 140 mg/mL, or 150 mg/mL of GX-H9.

According to the present invention, the formulation may be characterized by including 0.01% (w/v) to 0.2% (w/v) poloxamer 188, and may be preferably characterized by including 0.05% (w/v) to 0.15% (w/v), or 0.08% (w/v) to 0.12% (w/v), and more preferably 0.10% (w/v) poloxamer 188, but the present invention is not limited thereto.

According to the present invention, the formulation may be characterized by including 0.01% (w/v) to 0.2% (w/v) polysorbate 80, and may be preferably characterized by including 0.05% (w/v) to 0.15% (w/v), or 0.05% (w/v) to 0.10% (w/v), and more preferably 0.07% (w/v) polysorbate 80, but the present invention is not limited thereto.

According to the present invention, the formulation may be characterized by further including one or more excipients selected from the group consisting of histidine, arginine, glutamic acid, and sodium chloride (NaCl).

According to the present invention, the formulation may be characterized by further including a preservative.

According to the present invention, the preservative may be characterized by being any one or more selected from the group consisting of m-cresol, phenol, and benzyl alcohol, but the present invention is not limited thereto.

According to the present invention, the preservative may be characterized by being 1 mg/mL to 2 mg/mL of phenol, preferably 1 mg/mL of phenol, but the present invention is not limited thereto.

According to the present invention, the formulation may be characterized by including 1 mM to 20 mM histidine, preferably 5 mM to 15 mM, and more preferably 10 mM histidine, but the present invention is not limited thereto.

According to the present invention, the formulation may be characterized by including 40 mM to 70 mM arginine, preferably 50 mM to 60 mM, and more preferably 55 mM arginine, but the present invention is not limited thereto.

According to the present invention, the formulation may be characterized by including 40 mM to 70 mM glutamic acid, preferably 50 mM to 60 mM, and more preferably 55 mM glutamic acid, but the present invention is not limited thereto.

According to the present invention, the formulation may be characterized by including 80 mM to 100 mM sodium chloride, preferably 85 mM to 95 mM, and more preferably 90 mM sodium chloride, but the present invention is not limited thereto.

According to the present invention, the formulation may be characterized by having a pH of 5.5 to 7.0, preferably a pH of 5.9 to 6.9, more preferably a pH of 6.0 to 6.5, even more preferably a pH of 6.0 to 6.2, and most preferably a pH of 6.1, but the present invention is not limited thereto.

According to the present invention, the formulation may be characterized by forming less than 3.0%, preferably less than 2.0%, more preferably less than 1.5%, for example 0 to 1.5% of aggregates when stored in refrigerated conditions, for example, stored at 2°C to 8°C, preferably 4°C to 6°C, for approximately 6 months, but the present invention is not limited thereto.

According to the present invention, the formulation may be characterized by being administered subcutaneously or intramuscularly.

According to the present invention, the formulation may be characterized by being an injectable formulation.

According to the present invention, the formulation may be characterized by being stored in a container selected from the group consisting of a bottle, a microtube, a bag, a vial, a cartridge, an injector, and a syringe, but the present invention is not limited thereto.

For example, the formulation may be characterized by being stored in a container selected from the group consisting of a glass vial, a glass cartridge, a plastic cartridge, a prefilled syringe, a pen injector, and an autoinjector, but the present invention is not limited thereto.

The high-concentration GX-H9 pharmaceutical formulation according to the present invention may be administered to growth hormone-deficient adults or children. The high-concentration GX-H9 pharmaceutical formulation according to the present invention may be administered to a subject in various ways.

In the present invention, the subject may be a mammal, but the present invention is not limited thereto. Preferably, the subject may be a human.

For example, the formulation may be administered parenterally, for example subcutaneously or intramuscularly.

In the present invention, the term "pharmaceutical formulation" refers to a preparation that contains GX-H9 in a form that makes the biological activity of GX-H9 effective and does not contain more than an acceptable dose of ingredients that are toxic to the subject to whom the formulation is administered.

A "stable" formulation substantially retains the physiological and/or chemical stability and/or biological activity of GX-H9 during storage, management and distribution. In one aspect, the formulation substantially retains its biological activity as well as its physiological and chemical stability during storage. The storage period is generally selected based on the intended shelf life of the formulation. Stability may be measured at a selected temperature for a selected period of time. For example, in one aspect, the liquid formulation is stable at approximately 25°C for approximately 2 to 4 weeks, at least approximately 3 months, at least approximately 6 months, at least approximately 9 months, at least approximately 12 months, or at least approximately 18 months. As another example, the liquid formulation is stable at approximately 5°C for approximately 2 to 4 weeks, at least approximately 3 months, at least approximately 6 months, at least approximately 9 months, at least approximately 12 months, at least approximately 18 months, at least approximately 24 months, at least approximately 30 months, or at least approximately 36 months.

Analytical techniques for measuring protein stability are very diverse and are described in the related-art documents [Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993)].

The stability of the liquid formulation may be evaluated qualitatively and/or quantitatively in a variety of different ways, including evaluation of dimer, multimer and/or aggregate formation (for example, using liquid chromatography such as size exclusion ultra-high performance liquid chromatography (SE-UPLC), size exclusion high-performance liquid chromatography (SE-HPLC), reversed-phase ultra-high performance liquid chromatography (RP-UPLC), reversed-phase high-performance liquid chromatography (RP-HPLC), or the like; matrix-assisted laser desorption-ionization time-of-flight mass spectrometry (MALDI-TOF MS), analytical ultracentrifugation, light scattering (photon correlation spectroscopy, dynamic light scattering (DLS), static light scattering, multi-angle laser light scattering (MALLS), flow-based microscopic imaging, electronic impedance (Coulter) counting, light obscuration or other liquid particle counting systems, by measuring turbidity, and/or by visual inspection); by assessing charge heterogeneity using cation exchange chromatography (CEX), isoelectric focusing (IEF), for example, a capillary technique (cIEF), or capillary zone electrophoresis; amino-terminal or carboxy-terminal sequence analysis; mass spec peptide mapping (for example, using tryptic/LYS-C) analysis; evaluating the biological activity or antigen binding function of the antibody; and the like.

In the present invention, the pharmaceutical formulation may be an isotonic formulation, and the "isotonic" formulation has substantially the same osmotic pressure as human blood. Isotonic formulations will generally have an osmotic pressure of approximately 250 to 350 mOsm/kg. Isotonicity may be measured using, for example, a vapor pressure or ice-freezing type osmometer.

In the present invention, the "buffer" refers to a buffer that resists a change in pH by the action of its acid-base conjugate components. In some embodiments, the buffer of the present invention adjusts the pH of the formulation to a pH of approximately 5.0 to approximately 7.5, approximately 5.8 to approximately 7.0, approximately 6.0 to approximately 6.5, or approximately 6.0 to approximately 6.3. In one aspect, examples of buffers that control the pH alone or in combination include acetate, succinate, gluconate, histidine, citrate, phosphate, maleate, cacodylate, 2-[N-morpholino]ethanesulfonic acid (MES), bis(2-hydroxyethyl)iminotris[hydroxymethyl]methane (Bis-Tris), N-[2-acetamido]-2-iminodiacetic acid (ADA), glycylglycine and other organic acid buffers.

Meanwhile, in the present invention, a biological buffer, that is, a buffer known in the art to be used in biological systems or contexts thereof may be used as the buffer. For example, a buffer solution used in the present invention is a mixed buffer solution containing inorganic and organic salts.

Also, a preferred mixed buffer solution that can be used in the present invention is a biological buffer solution and may include amino acids. Preferred amino acids that can be used in the present invention may be one or more selected from the group consisting of histidine, arginine, and glutamic acid, and preferably include all of histidine, arginine, and glutamic acid.

In the present invention, the "surfactant" refers to an agent that lowers the surface tension of a liquid. In one aspect, the surfactant may be a nonionic surfactant. In a preferred aspect, the present invention may include both polysorbate 80 and poloxamer 188 as surfactants, but the present invention is not limited thereto. Examples of surfactants that can be further included in the present invention include any one or more selected from the group consisting of polysorbate (polyoxyethylene sorbitan monolaurate, for example, polysorbate 20); TRITON (t-octylphenoxypolyethoxyethanol, nonionic detergents, Union Carbide, a subsidiary of Dow Chemical Co., Midland Mich.); sodium dodecyl sulfate (SDS); sodium laurel sulfate; sodium octyl glycoside; lauryl-, myristyl-, linoleyl-, or stearyl-sulfobetaine; lauryl-, myristyl-, linoleyl- or stearyl-sarcosine; linoleyl-, myristyl-, or cetyl-betaine; lauroamidopropyl-, cocamidopropyl-, linoleamidopropyl-, myristamidopropyl-, palmidopropyl-, or isostearamidopropyl-betaine (e.g., lauroamidopropyl); myristamidopropyl-, palmidopropyl-, or isostearamidopropyl-dimethylamine; sodium methyl cocoyl-, or disodium methyl oleyl-taurate; sorbitan monopalmitate; and the MONAQUAT series (Mona Industries, Inc., Paterson, N.J.); polyethylene glycol (PEG), polypropylene glycol (PPG), and copolymers of poloxyethylene and poloxypropylene glycol (e.g., Pluronics/Poloxamer, PF68, and the like); and the like.

In one embodiment, the formulation according to the present invention is sterile and contains no preservatives. In another embodiment, the formulation according to the present invention may include any preservative, and the preservative may be a paraben-free preservative. Parabens are a series of parahydroxybenzoates or esters of parahydroxybenzoic acid, which cause cytokine release and stimulation and are known to be associated with several types of cancer. Examples of parabens include methyl paraben, ethyl paraben, propyl paraben, butyl paraben, heptyl paraben, isobutyl paraben, isopropyl paraben, benzyl paraben, and sodium salts thereof.

Exemplary paraben-free preservatives include methylphenol (cresol), such as 3-methylphenol (meta-cresol or m-cresol), phenol, phenethyl alcohol, caprylyl glycol, phenoxyethanol, sorbate, potassium sorbate, sodium sorbate, sorbic acid, sodium benzoate, benzoic acid, acemannan, oleuropein, carvacrol, a cranberry extract, gloconolactone, a green tea extract, *Helianthus annuus* seed oil, a *Lactobacillus* fermentation product, an *Usnea barbata* extract, polyaminopropyl biguanide, polyglyceryl-3 palmitate, polyglyceryl-6 caprylate, a pomegranate extract, a *Populus tremuloides* bark extract, resveratrol, a *Rosmarinus officinalis* leaf extract, benzyl alcohol, or any combination thereof.

The formulation according to the present invention may further include an antioxidant. The term "antioxidant" refers to an agent that inhibits the oxidation of other molecules. In the present invention, examples of antioxidants include citrate, lipoic acid, uric acid, glutathione, tocopherol, carotene, lycopene, cysteine, phosphonate compounds (e.g., etidronic acid), desferoxamine, and malate.

GX-H9 used in the present invention may be substantially pure (*i.e.,* free of contaminating proteins and the like) and may be substantially homogeneous. "Substantially pure" GX-H9 means that it is included in an amount of at least approximately 90% by weight, alternatively at least approximately 95 or 97% by weight, based on the total weight of proteins in the pharmaceutical formulation.

GX-H9 may include "substantially homologous" GX-H9, and the term "substantially homologous" GX-H9 means that it has mutations and equivalent activity to the GX-H9 used in the present invention. In one aspect, the term substantially homogeneous may mean substantially the same as an amino acid sequence of GX-H9, and the substantially identical sequence is aligned to correspond as much as possible to the GX-H9 amino acid sequence of the present invention and any other sequences. In this case, when the aligned sequences are analyzed using algorithms commonly used in the art, the substantially identical sequence refers to a sequence having a homology of at least 90%, most preferably a homology of at least 95%, 96% or more, 97% or more, 98% or more, or 99% or more.

### [Mode for Invention]

Hereinafter, the present invention will be described in more detail with reference to examples thereof. However, it will be obvious to those skilled in the art that the following examples are provided only for illustration of the present invention and should not be construed as limiting the scope of the present invention.

### Example 1: Evaluation of 12-week stability of high-concentration formulation of hGH-hyFc

### 1-1. Summary of preliminary experimental results

Preliminary experiments using high-dose formulations of hGH-hyFc (hereinafter referred to interchangeably as "GX-H9") showed greater aggregation resistance to temperature stress over the pH range of 6.0 to 6.5 compared to other pH ranges. Also, when a histidine buffering agent was used, a higher level of stability was confirmed compared to a phosphate buffering agent. Meanwhile, high stability was achievable when poloxamer 188 and polysorbate 80 were used under freezing/thawing and stirring conditions.

Overall, the SE-HPLC results showed higher stability at pH 6.1, while the RP-UPLC results showed higher stability in the pH 6.5 formulation containing sodium chloride (data not shown).

### 1-2. Experimental design

Hereinafter, the stabilities of a total of five different formulations, which further include a phosphate buffer pH 6.9 formulation, were analyzed according to temperature (-70°C, 2 to 8°C, 25°C, 45°C) stress, freezing/thawing stress, and light shaking stress. Table 1 lists the formulation conditions for GX-H9 and the concentration and type of excipients.

**[Table 1]**

| **pH** | Concentration **(mg/mL)** | **10 mM** Histidine | **15 mM** Sodium phosphate | Arginine **(mM)** | Glutamic acid **(mM)** | Sodium chloride **(mM)** | Sucrose **% (w/v**) | **0.1% (w/v)** Poloxamer **188** |
|---|---|---|---|---|---|---|---|---|
| **6.1** | 150 | + | | 55 | 55 | 90 | | + |
| **6.1** | 100 | + | | 55 | 55 | 90 | | + |
| **6.5** | 150 | + | | | | 80 | 4 | + |
| **6.9** | 150 | | + | | | | 200 mM | + |
| **6.9** | 30 | | + | | | 50 | 5 | + |

A solution including GX-H9 was subjected to ultrafiltration (UF)/diafiltration (DF) using each buffer under the formulation conditions (excluding poloxamer 188) described in Table 1. After it was confirmed that the buffer sufficiently reached equilibrium by measuring pH and conductivity, the solution was concentrated to the desired concentration and poloxamer 188 was added to adjust the target concentration.

Under sterile conditions, GX-H9 samples made from the individual formulations were filtered through a 0.2 µm polyvinylidene difluoride (PVDF) membrane, dispensed into pre-sterilized depyrogenated borosilicate vials, and then capped. Thereafter, the vials were crimped with an aluminum seal. Then, each of the vials was placed under stress conditions for stability testing, and samples were extracted at the desired time points to perform stability evaluation (Table 2). Aliquots of the samples were diluted as necessary for proper analysis, and the remaining samples were maintained at -70°C for backup testing. Table 2 shows the stress conditions and the experimental conditions for analysis items.

**[Table 2]**

| Conditions | Sampling time | **A280 nm,** Cuvette | **A330 nm,** Diluted | Visual appearance (liquid phase) | **RP-UPLC** | **SE-U/HPLC** | **pH** |
|---|---|---|---|---|---|---|---|
| **-70 °C** | T=0 | 1 | 1 | 1 | 1 | 1 | 1 |
| 3-Day shaking stress **(2-8 °C)** | 3 days | 1 | 1 | 1 | 1 | 1 | 1 |
| 5-Cycle freezing(-70°C) /Thawing (ambient) | 1 week | 1 | 1 | 1 | 1 | 1 | 1 |
| **2-8 °C** | 1 week | 1 | 1 | 1 | 1 | 1 | 1 |
| **25 °C** | 1 week | 1 | 1 | 1 | 1 | 1 | 1 |
| **45 °C** | 1 week | 1 | 1 | 1 | 1 | 1 | 1 |
| **2.8 °C** | 3 weeks | 1 | 1 | 1 | 1 | 1 | 1 |
| **25 °C** | 3 weeks | 1 | 1 | 1 | 1 | 1 | 1 |
| **45 °C** | 3 weeks | 1 | 1 | 1 | 1 | 1 | 1 |
| **-70 °C** | 6 weeks | 1 | 1 | 1 | 1 | 1 | 1 |
| **2-8** °**C** | 6 weeks | 1 | 1 | 1 | 1 | 1 | 1 |
| **25 °C** | 6 weeks | 1 | 1 | 1 | 1 | 1 | 1 |
| **-70 °C** | 12 weeks | 1 | 1 | 1 | 1 | 1 | 1 |
| **2-8 °C** | 12 weeks | 1 | 1 | 1 | 1 | 1 | 1 |
| **25 °C** | 12 weeks | 1 | 1 | 1 | 1 | 1 | 1 |

### 1-3. Concentration (A280), turbidity (A330), appearance, and pH changes

The concentration, turbidity, visual appearance, and pH changes of each formulation were determined under stress conditions.

The concentration of the sample was determined by absorbance measurements at 280 nm using an extinction coefficient of 1.0 mg/mL = 1.04 AU. The final protein concentration identified was corrected for scattering ((A280-A330)/ε₂₈₀ = Corrected protein concentration).

The results of confirming the protein concentration, turbidity, appearance, and pH of the samples after various stress conditions are shown in Tables 3 and 4. No clear differences were observed with respect to the protein concentration, turbidity, and pH under different stress conditions. No visible particulates were observed in all the formulations under various stress conditions. As a result of 5 freezing/thawing cycles or 3-day shaking stress at 2 to 8°C, there were no discernible analytical changes in any formulations.

Table 3 lists the concentration, turbidity, appearance, and pH changes after temperature stress.

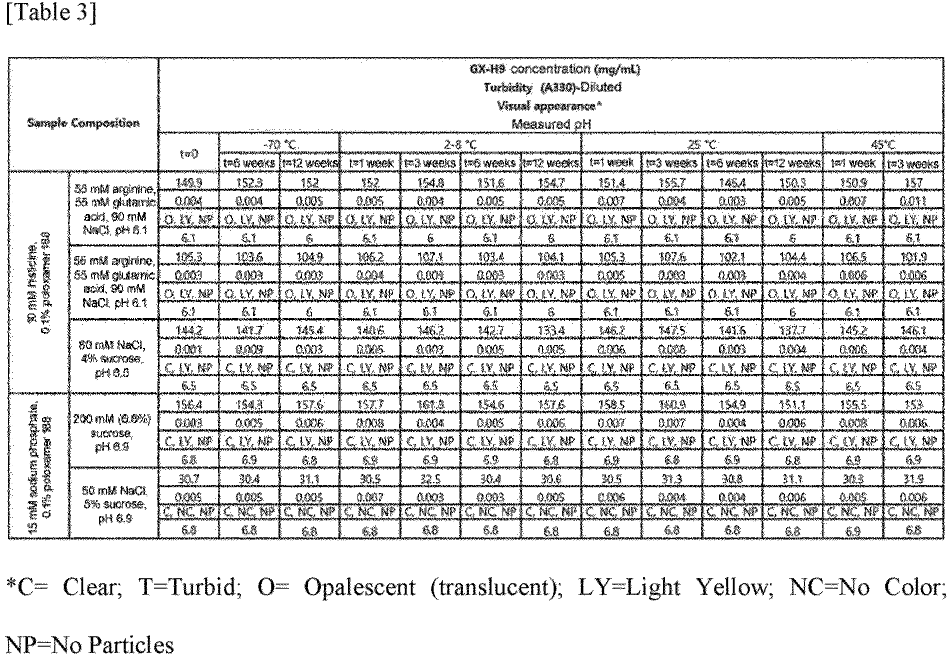

Table 4 lists the concentration, turbidity, appearance, and pH changes after freezing/thawing and shaking stress.

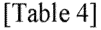

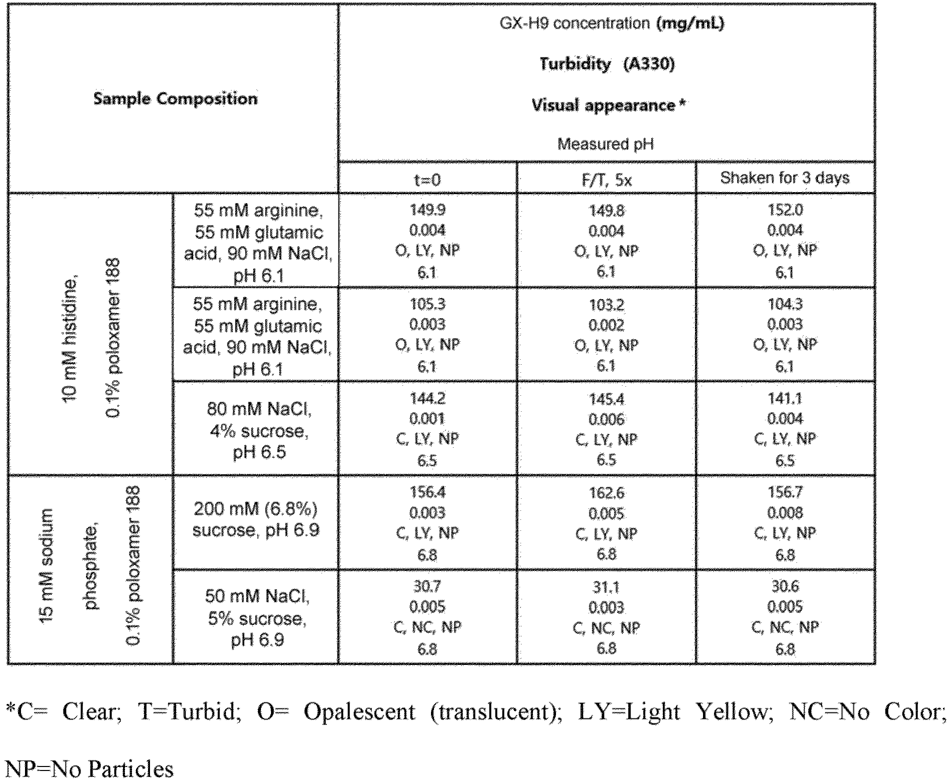

The target concentration of GX-H9 was achieved in all the tested formulations, which resulted in scattering levels of less than 1% for most formulations after preparation. After the stress response, the protein concentration, turbidity, appearance, and pH of the samples were checked. As a result, no clear differences were observed with respect to the protein concentration, turbidity, and pH under various stress conditions in all the tested formulations.

### 1-4. SE-UPLC

SE-UPLC (also known as gel filtration chromatography) separates the molecular forms of proteins based on their sizes. In this method, high-molecular species (e.g., aggregates, IgG dimers and oligomers) are eluted as pre-peaks prior to the desired (monomeric) IgG species. Low-molecular species (e.g., degradation products and fragments) are then eluted as post-peaks.

SE-UPLC was performed using Waters Acquity UPLC BEH SEC, 200A (4.6 x 300 mm, 1.7 µm) and a mobile phase of 100 mM sodium phosphate and 200 mM arginine-HCl, pH 7.0.

Table 5 lists the SE-UPLC results according to temperature stress.

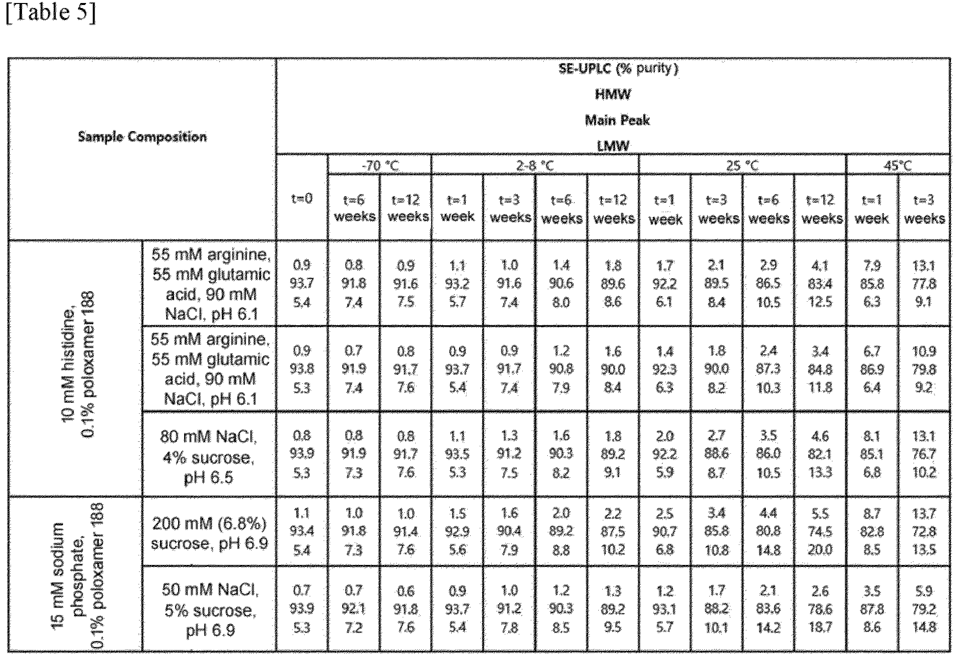

Table 6 lists the SE-UPLC results according to the freezing/thawing and shaking stress.

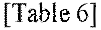

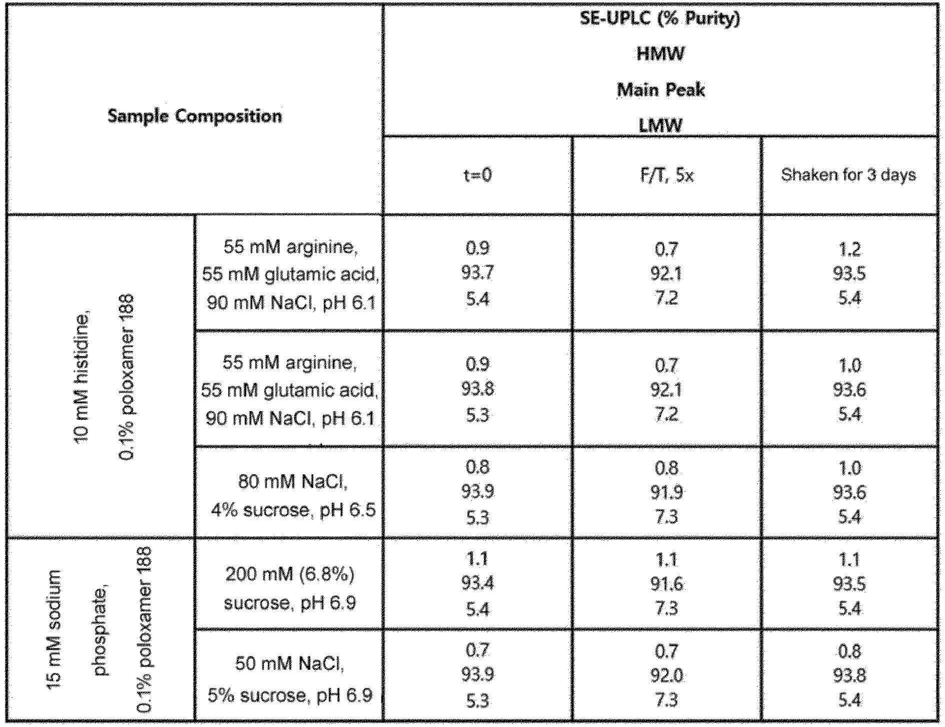

As shown in Tables 5 and 6, the SE-UPLC results of the samples stored at -70°C, 2 to 8°C, 25°C, and 45°C for 12 weeks showed that both the high-molecular weight (HMW) and low-molecular weight (LMW) species increased. Comparison results of stability endpoints showed that the GX-H9 stability was higher at pH 6.1 compared to higher pH values. Meanwhile, among the same pH 6.1 formulations, the 100 mg/mL formulation was more stable than the 150 mg/mL formulation. No discernible trends were observed between all the tested formulations when exposed to freezing/thawing and shaking stress at 2 to 8°C. This was because the resulting data was similar within the t = 0 results and test variance (Tables 5 and 6).

### 1-5. RP-UPLC

RP-UPLC separates molecules based on differences in hydrophobicity. Separation depends on the binding of solutes in a mobile phase to immobilized hydrophobic ligands in a stationary phase. Elution is usually accomplished by changing the hydrophobicity of a mobile phase through organic solvents in order of increasing molecular hydrophobicity. Solvent gradient separation was carried out using an ultra-high performance liquid chromatography system (Waters Acquity H-Class UPLC) equipped with a UV detector (A220) monitoring at 220 nm and integrated software (Chromeleon 7.2) along with a Waters Acquity UPLC Protein BEH300 C4 column (2.1 x 150 mM).

Table 7 lists the RP-UPLC results according to the temperature stress.

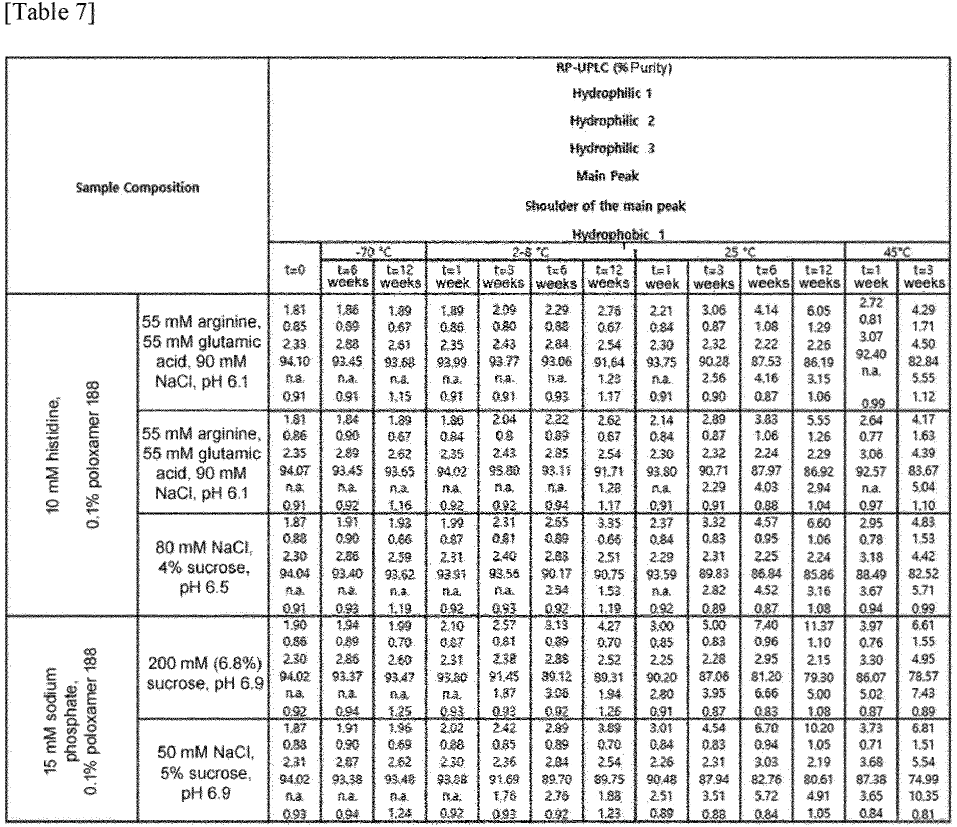

Table 8 lists the RP-UPLC results according to the freezing/thawing and shaking stress.

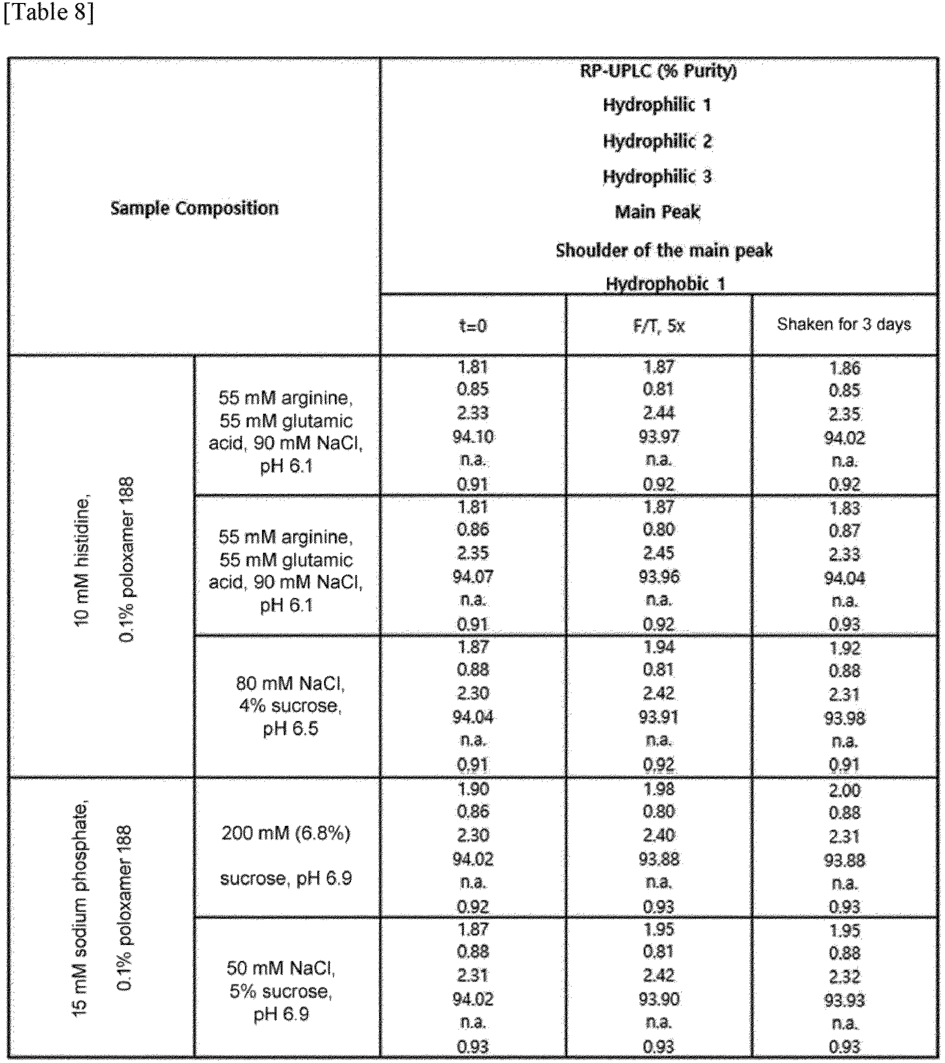

The RP-UPLC results of the samples stored at -70°C, 2 to 8°C, and 25°C for 12 weeks or at 45°C for 3 weeks showed that the pH 6.1 formulation containing arginine and glutamic acid had better stability compared to other high pH formulations. No discernible trends were observed between all the tested formulations when exposed to freezing/thawing and shaking stress at 2 to 8°C. This was because the resulting data was similar within the t = 0 results and test variance (Tables 7 and 8).

### Example 2: Evaluation of stability of hGH-hyFc formulation with and without polysorbate 80

Based on the facts that even when 10 mM histidine, 0.1% (w/v) poloxamer 188, 55 mM arginine, 55 mM glutamic acid, and 90 mM NaCl (pH 6.1) selected for the best formulation in Example 1 were used, the best formulation showed a tendency to produce insoluble foreign substances and increase fine particles, and thus the hGH of GX-H9 had many hydrophobic patches so that proteins themselves could form aggregates by hydrophobic interactions, and that the proteins were adsorbed to the gas/oil/solid surfaces in a DP container to form a kind of protein film, and patches made by the detachment of proteins could form aggregates with insoluble particles, the present inventors made attempts to confirm that the aggregate formation was significantly improved in a liquid formulation to which polysorbate 80 and poloxamer 188 were added together as surfactants in order to develop a formulation capable of preventing the formation of such aggregates.

Based on the results showing that the production of insoluble foreign substances and fine particles was better inhibited when poloxamer 188 and polysorbate 80 were used together compared to when poloxamer 188 was used alone, appearance analysis, fine particle analysis, SE-UPLC analysis, and reversed-phase high performance liquid chromatography (RP-HPLC) analysis were performed on a total of eight formulations, which were prepared by adding 0.03% (w/v) polysorbate 80 to four formulation compositions including 60 mg/mL of hGH-hyFc in a state in which the concentration of poloxamer 188 was fixed at 0.10% (w/v).

### 2-1. Appearance analysis (visual inspection of insoluble foreign substance)

Various formulation compositions were prepared using an hGH-hyFc stock solution (drug substance (DS)), filtered with a 0.22 µm PES filter in a biosafety cabinet, and then dispensed into glass vials in an amount of approximately 0.8 mL to prevent external dust and the like from being included therein. Sample containers stored in refrigerated conditions (5°C) and at room temperature (25°C) were taken out, and adjusted to room temperature for approximately 30 minutes. Then, the outside surfaces of the containers were cleaned with alcohol and the like on a foreign substance inspection table, and the state of the samples in the containers was then directly observed with the naked eye to check for the presence of insoluble foreign substances for six months.

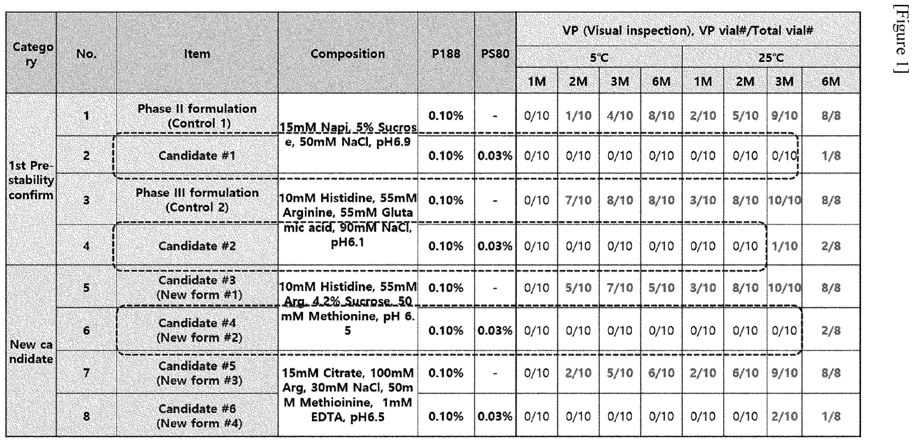

As a result, as shown in FIG. 1, when the sample was stored in refrigerated conditions (5°C), no insoluble foreign substances were formed for 6 months under the polysorbate 80-containing conditions, but the production of insoluble foreign substances was observed in a significant number of the 10 samples when the samples were stored in refrigerated conditions for 6 months under the polysorbate 80-free conditions. Meanwhile, when the samples were stored at room temperature (25°C), it was confirmed that the production of insoluble foreign substances was generally inhibited under the polysorbate 80-containing conditions compared to the polysorbate 80-free conditions. In particular, Candidates #1 and #4 did not produce insoluble foreign substances for 3 months. However, the production of insoluble foreign substances was observed in all the vials when the samples were stored at room temperature for 6 months under the polysorbate 80-free formulation conditions.

### 2-2. Fine particle (sub-visible particle) analysis

One mL of each sample was prepared for each condition in a glass vial, and fine particle analysis was performed using Microflow Imaging equipment (MFI 5200, Proteinsimple), MFI View System Software (MVSS) Version 2-R4.1.0.40.4816, and MFI View Analysis Suite (MVAS) Version 1.4.0 by commissioning external companies.

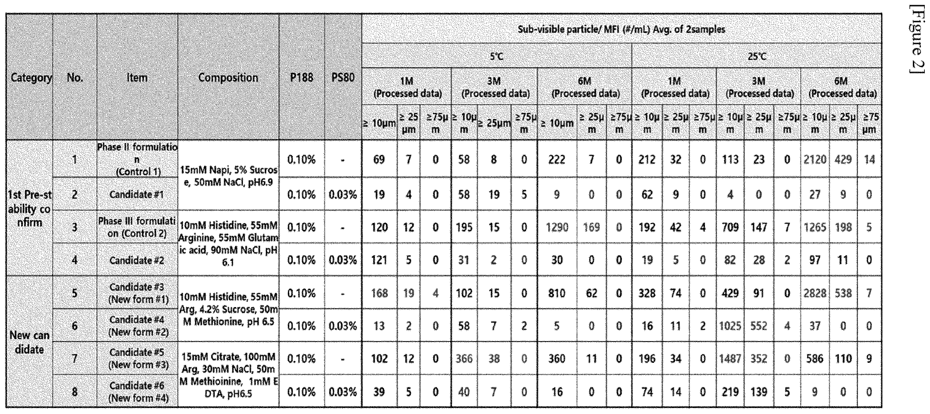

As a result, as shown in FIG. 2, it was observed that Candidates #1, #2, #4, and #6 containing polysorbate 80 well inhibited the production of fine particles when stored in refrigerated conditions (5°C) for 6 months. In relation to the production of fine particles, it can be seen that significantly higher levels of fine particles were produced in the formulations that did not contain polysorbate 80 both in refrigerated conditions and at room temperature.

### 2-3. SE-UPLC analysis

Mobile phase solutions of 100 mM sodium phosphate (pH 7.0) and 200 mM arginine-HCl were allowed to flow at a rate of 0.25 mL/min using an ACQUITY UPLC Protein BEH200 (4.6*300 mm) (Waters, 186005226) column, and 10 µg of each sample was injected for each condition to confirm the peak of the GX-H9 protein at an absorbance of 280 nm.

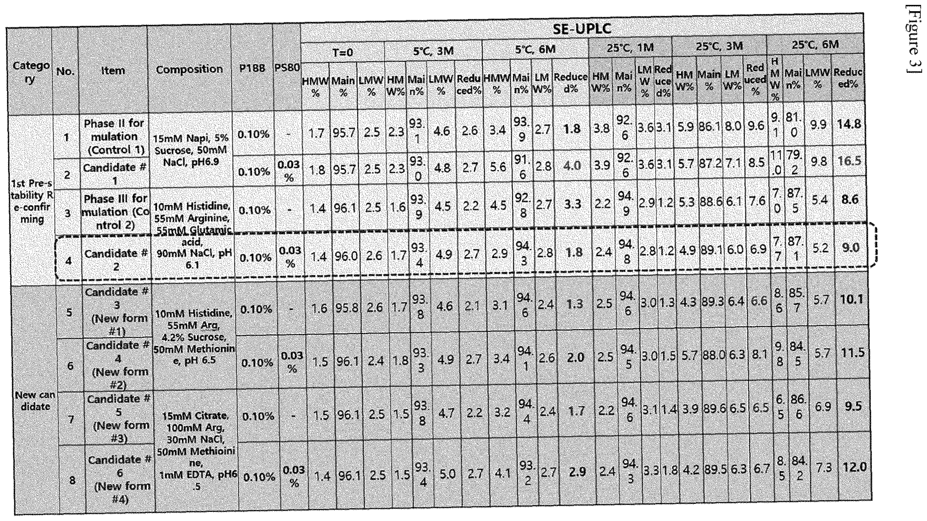

As a result, as shown in FIG. 3, it was confirmed that Candidates #2, #3, and #5 have excellent purity when stored in refrigerated conditions (5°C) and at room temperature (25°C) for 6 months. In particular, as the formulation containing polysorbate 80, Candidate #2 had the highest purity. It was confirmed that Candidates #3 and #5, which are formulations that do not contain polysorbate 80, had a similar purity compared to Candidate #2 when stored in refrigerated conditions for 6 months, but had a relatively low purity when stored at room temperature for 6 months.

### 2-4. RP-HPLC

Mobile phase solutions of 0.05% trifluoroacetic acid (TFA) in water (MPA) and 0.05% trifluoroacetic acid (TFA) in acetonitrile (MPB) were allowed to flow at a rate of 0.5 mL/min under gradient conditions in Table 9 using a Proteonavi C4 300A 5 µm (Shiseido, 80205) column, and 20 µg of each sample was injected to confirm the peak of the GX-H9 protein at an absorbance of 220 nm.

**[Table 9]**

| **Gradient** | | | |
|---|---|---|---|
| Time | Flow | % MPA | % MPB |
| Initial | 0.5 | 49.0 | 51.0 |
| 30.0 | 0.5 | 30.0 | 70.0 |
| 30.5 | 0.5 | 0.0 | 100.0 |
| 31.0 | 0.5 | 49.0 | 51.0 |
| 40.0 | 0.5 | 49.0 | 51.0 |

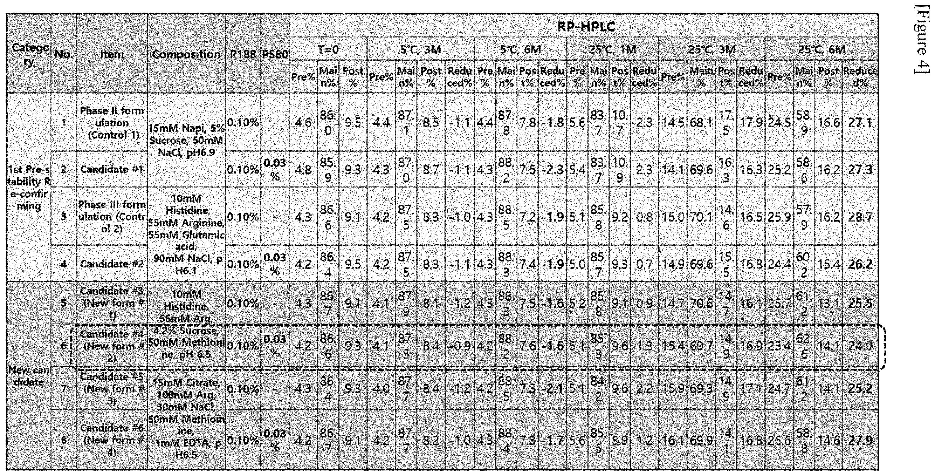

As a result, as shown in FIG. 4, all the formulations had a similar purity when stored in refrigerated conditions (5°C) for 6 months, but Candidate #4 showed the relatively best purity when stored at room temperature (25°C) for 6 months.

### Example 3: Evaluation of stability of hGH-hyFc formulation according to content of polysorbate 80

The formulation stability of hGH-hyFc (appearance analysis, fine particle analysis, SE-UPLC analysis, RP-HPLC analysis) was tested by the same experimental method as in Example 2 by varying the concentration of polysorbate 80 in Candidate #2, which was judged to be the most suitable formulation based on the results of Example 2.

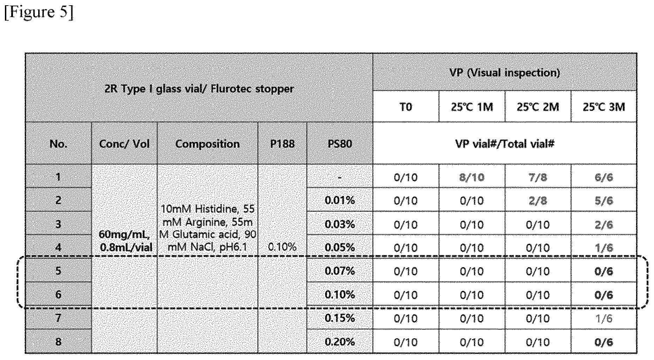

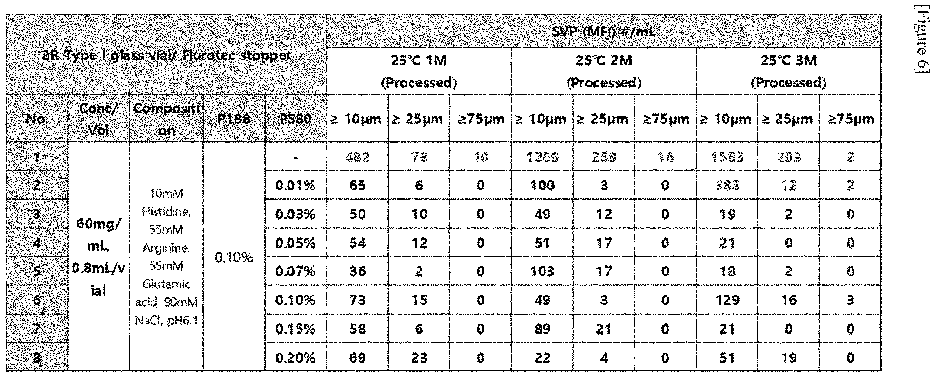

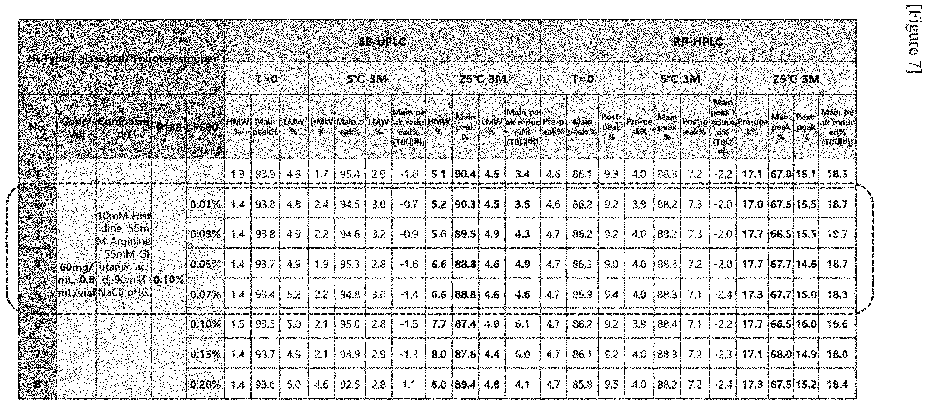

As a result, it can be seen that the formulation further including at least 0.07% (w/v) polysorbate 80 was suitable for storage at room temperature (25°C) for 3 months or more in the case of the appearance analysis (FIG. 5). Also, in the fine particle analysis, generally similar levels of fine particles were observed in the polysorbate 80 concentration range of 0.03 to 0.15% (w/v) (FIG. 6). The SE-UPLC and RP-HPLC results showed that there was no significant difference due to the concentration of polysorbate 80 when the samples were stored in refrigerated conditions (5°C), the SE-UPLC results showed that, when the samples were stored at room temperature, the purity decreased when the concentration of polysorbate 80 was 0.10% (w /v) or more, and the RP-HPLC results showed that there was no significant difference in purity due to the concentration of polysorbate 80 when the samples were stored at room temperature (FIG. 7).

### Example 4: Evaluation of stability of high-concentration hGH-hyFc formulation

Summarizing the above examples, (i) when hGH-hyFc was treated at 60, 80, 100, and 120 mg/mL, respectively, while polysorbate 80 was fixed at 0.07% (w/v), and the samples were stored in glass cartridges, (ii) when polysorbate 80 was treated at 0.03, 0.05, 0.07, 0.10, and 0.15% (w/v), respectively, while hGH-hyFc was fixed at 120 mg/mL, and the samples were stored in glass cartridges, (iii) when preservatives such as m-cresol, phenol, or benzyl alcohol were treated while hGH-hyFc and polysorbate 80 were fixed at 60 mg/mL and 0.07% (w/v), respectively, and the samples were stored in glass vials, it was confirmed through appearance analysis whether the insoluble foreign substances were observed when the samples were stored in refrigerated conditions (5°C) and at room temperature (25°C). Then, formulation stability was evaluated by confirming a change in purity.

### 4-1. Appearance analysis (visual inspection of insoluble foreign substances)

Various formulation compositions were prepared using an hGH-hyFc stock solution (DS), filtered with a 0.22 µm PES filter in a biosafety cabinet, and then dispensed into glass vials in an amount of approximately 1.0 mL to prevent external dust and the like from being included therein. Sample containers stored in refrigerated conditions (5°C) and at room temperature (25°C) were taken out, and adjusted to room temperature for approximately 30 minutes. Then, the outside surfaces of the containers were cleaned with alcohol and the like on a foreign substance inspection table, and the state of the samples in the containers was then directly observed with the naked eye to check for the presence of insoluble foreign substances for six months.

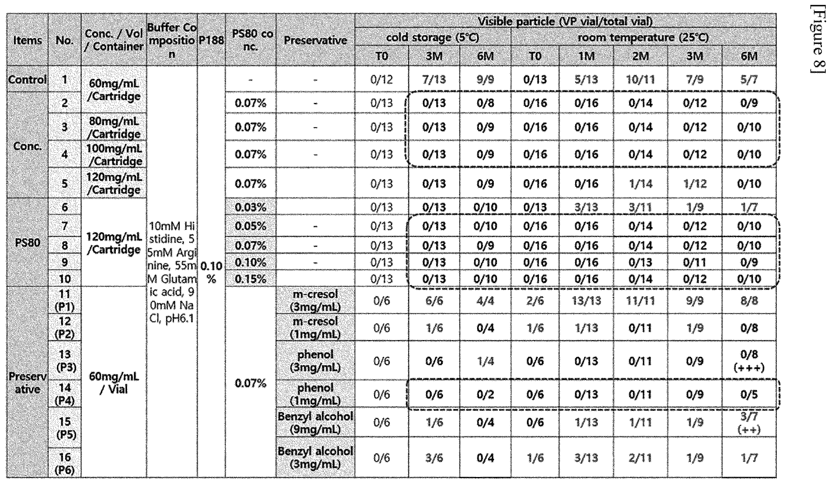

As a result, (i) when hGH-hyFc was treated at 60 to 100 mg/mL while polysorbate 80 was fixed at 0.07% (w/v), and the samples were stored in glass cartridges, it was confirmed that insoluble foreign substances were not produced in refrigerated conditions and at room temperature for 6 months, (ii) when polysorbate 80 was treated at 0.05 to 0.15% (w/v) while hGH-hyFc was fixed at 120 mg/mL, and the samples were stored in glass cartridges, it was confirmed that insoluble foreign substances were not produced in refrigerated conditions and at room temperature for 6 months, and (iii) when 1 mg/mL of phenol as a preservative was treated while hGH-hyFc and polysorbate 80 were fixed at 60 mg/mL and 0.07% (w/v), respectively, and the samples were stored in glass vials, it was confirmed that insoluble foreign substances were not produced in refrigerated conditions and at room temperature for 6 months (FIG. 8).

### 4-2. SE-UPLC and RP-HPLC analysis

Samples for each condition were taken from glass cartridges or glass vials and diluted to 2 mg/mL using a mobile phase buffer and a formulation buffer. Thereafter, the diluted samples were filtered through a 0.22 µm cellulose acetate centrifugal tube filter, placed in LC vials, and then analyzed by SE-UPLC or RP- HPLC.

For SE-UPLC, mobile phase solutions of 100 mM sodium phosphate (pH 7.0) and 200 mM arginine-HCl were allowed to flow at a rate of 0.25 mL/min using an ACQUITY UPLC Protein BEH200 (4.6*300 mm) (Waters, 186005226) column, and 10 µg of each sample was injected to confirm the peak of the GX-H9 protein at an absorbance of 280 nm.

For RP-HPLC, mobile phase solutions of 0.05% trifluoroacetic acid (TFA) in water (MPA) and 0.05% trifluoroacetic acid (TFA) in acetonitrile (MPB) were allowed to flow at a rate of 0.5 mL/min under gradient conditions in Table 9 using a Proteonavi C4 300Å 5µm (Shiseido, 80205) column, and 20 µg of each sample was injected to confirm the peak of the GX-H9 protein at an absorbance of 220 nm.

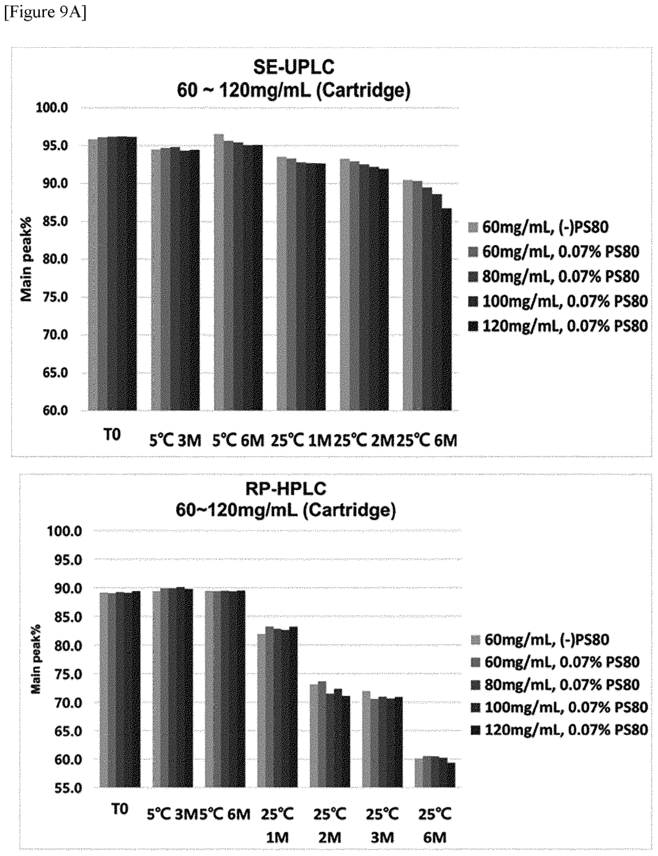

As a result, (i) when hGH-hyFc was treated at 60 to 120 mg/mL while polysorbate 80 was fixed at 0.07% (w/v), and the samples were stored in glass cartridges, there was a slight difference (0.5%) in purity between hGH-hyFc doses of 60 mg/mL and 120 mg/mL in SE-UPLC when the samples were stored in refrigerated conditions (5°C), but the difference in purity between the hGH-hyFc doses of 60 mg/mL and 120 mg/mL was confirmed to be 3.6% when the samples were stored at room temperature (25°C). Meanwhile, when the samples were stored at room temperature, the difference in purity between the hGH-hyFc doses of 60 mg/mL and 120 mg/mL was slight at 0.8% (FIG. 9A).

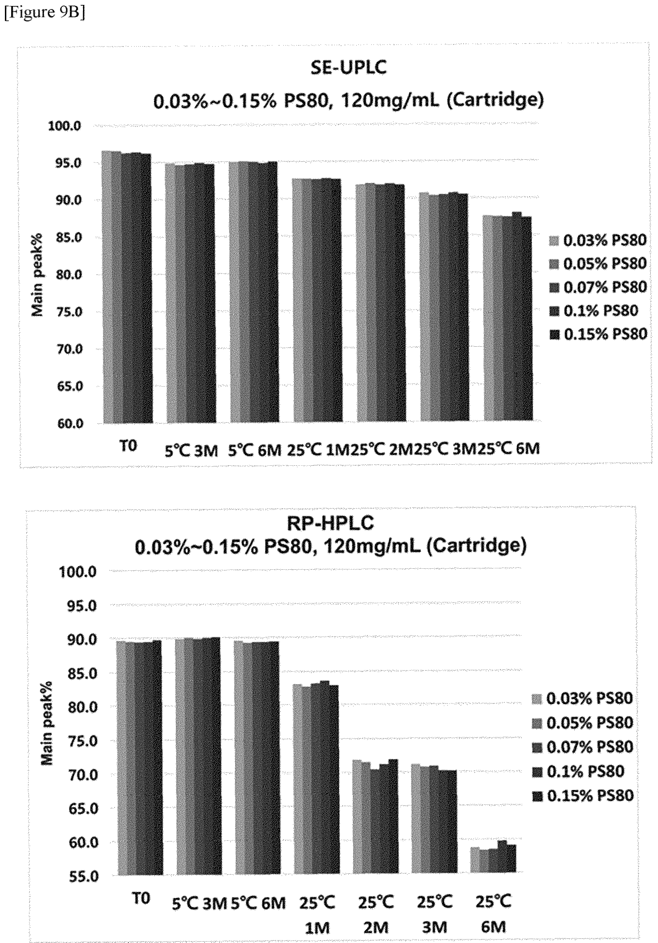

(ii) when polysorbate 80 was treated at 0.03 to 0.15% (w/v) while hGH-hyFc was fixed at 120 mg/mL, and the samples were stored in glass cartridges, there was no difference in purity between polysorbate 80 concentrations of 0.03% (w/v) and 0.15% (w/v) when the samples were stored in refrigerated conditions, and there was a slight difference in purity between the polysorbate 80 concentrations of 0.03% (w/v) and 0.15% (w/v) when the samples were stored at room temperature (FIG. 9B).

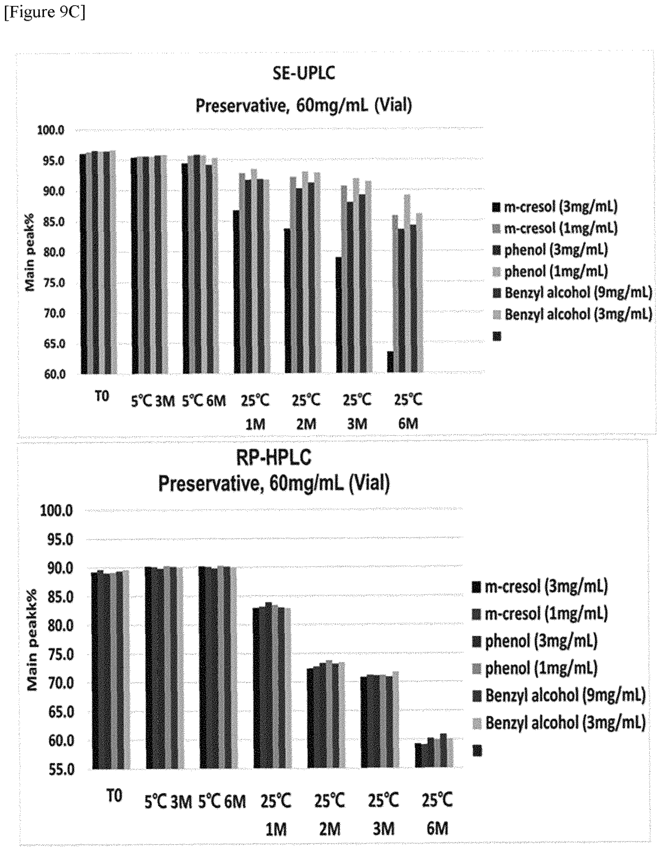

(iii) when preservatives such as m-cresol, phenol, or benzyl alcohol was treated while hGH-hyFc and polysorbate 80 were fixed at 60 mg/mL and 0.07% (w/v), respectively, and the samples were stored in glass vials, the SE-UPLC results showed that the smallest decrease in purity was observed under the 1 mg/mL phenol-containing conditions, and the RP-HPLC results showed that there was no significant difference in purity for all the preservatives (FIG. 9C).

Although specific parts of the present invention have been described in detail, it will be obvious to a person with ordinary skill in the art that such a specific description is merely a preferred embodiment and the scope of the present invention is not limited thereby. Accordingly, the actual scope of the present invention will be defined by the appended claims and equivalents thereof.

### [Sequence List Text]

1. GX-H9 (hGH-hyFc5) 
Phe Pro Thr Ile Pro Leu Ser Arg Leu Phe Asp Asn Ala Met Leu Arg

Ala His Arg Leu His Gln Leu Ala Phe Asp Thr Tyr Gln Glu Phe Glu

Glu Ala Tyr Ile Pro Lys Glu Gln Lys Tyr Ser Phe Leu Gln Asn Pro

Gln Thr Ser Leu Cys Phe Ser Glu Ser Ile Pro Thr Pro Ser Asn Arg

Glu Glu Thr Gln Gln Lys Ser Asn Leu Glu Leu Leu Arg Ile Ser Leu

Leu Leu Ile Gln Ser Trp Leu Glu Pro Val Gln Phe Leu Arg Ser Val
 
Phe Ala Asn Ser Leu Val Tyr Gly Ala Ser Asp Ser Asn Val Tyr Asp

Leu Leu Lys Asp Leu Glu Glu Gly Ile Gln Thr Leu Met Gly Arg Leu

Glu Asp Gly Ser Pro Arg Thr Gly Gln Ile Phe Lys Gln Thr Tyr Ser

Lys Phe Asp Thr Asn Ser His Asn Asp Asp Ala Leu Leu Lys Asn Tyr

Gly Leu Leu Tyr Cys Phe Arg Lys Asp Met Asp Lys Val Glu Thr Phe

Leu Arg Ile Val Gln Cys Arg Ser Val Glu Gly Ser Cys Gly Phe Arg

Asn Thr Gly Arg Gly Gly Glu Glu Lys Lys Lys Glu Lys Glu Lys Glu

Glu Gln Glu Glu Arg Glu Thr Lys Thr Pro Glu Cys Pro Ser His Thr

Gln Pro Leu Gly Val Phe Leu Phe Pro Pro Lys Pro Lys Asp Thr Leu

Met Ile Ser Arg Thr Pro Glu Val Thr Cys Val Val Val Asp Val Ser

Gln Glu Asp Pro Glu Val Gln Phe Asn Trp Tyr Val Asp Gly Val Glu

Val His Asn Ala Lys Thr Lys Pro Arg Glu Glu Gln Phe Asn Ser Thr

Tyr Arg Val Val Ser Val Leu Thr Val Leu His Gln Asp Trp Leu Asn

Gly Lys Glu Tyr Lys Cys Lys Val Ser Asn Lys Gly Leu Pro Ser Ser

Ile Glu Lys Thr Ile Ser Lys Ala Lys Gly Gln Pro Arg Glu Pro Gln

Val Tyr Thr Leu Pro Pro Ser Gln Glu Glu Met Thr Lys Asn Gln Val

Ser Leu Thr Cys Leu Val Lys Gly Phe Tyr Pro Ser Asp Ile Ala Val

Glu Trp Glu Ser Asn Gly Gln Pro Glu Asn Asn Tyr Lys Thr Thr Pro

Pro Val Leu Asp Ser Asp Gly Ser Phe Phe Leu Tyr Ser Arg Leu Thr

Val Asp Lys Ser Arg Trp Gln Glu Gly Asn Val Phe Ser Cys Ser Val

Met His Glu Ala Leu His Asn His Tyr Thr Gln Lys Ser Leu Ser Leu

Ser Leu Gly Lys
2. hGH (GenBank: AAA98618.1) (excluding a signal sequence)
3. hyFc5

Arg Asn Thr Gly Arg Gly Gly Glu Glu Lys Lys Lys Glu Lys Glu Lys

Glu Glu Gln Glu Glu Arg Glu Thr Lys Thr Pro Glu Cys Pro Ser His

Thr Gln Pro Leu Gly Val Phe Leu Phe Pro Pro Lys Pro Lys Asp Thr

Leu Met Ile Ser Arg Thr Pro Glu Val Thr Cys Val Val Val Asp Val

Ser Gln Glu Asp Pro Glu Val Gln Phe Asn Trp Tyr Val Asp Gly Val

Glu Val His Asn Ala Lys Thr Lys Pro Arg Glu Glu Gln Phe Asn Ser

Thr Tyr Arg Val Val Ser Val Leu Thr Val Leu His Gln Asp Trp Leu

Asn Gly Lys Glu Tyr Lys Cys Lys Val Ser Asn Lys Gly Leu Pro Ser

Ser Ile Glu Lys Thr Ile Ser Lys Ala Lys Gly Gln Pro Arg Glu Pro

Gln Val Tyr Thr Leu Pro Pro Ser Gln Glu Glu Met Thr Lys Asn Gln

Val Ser Leu Thr Cys Leu Val Lys Gly Phe Tyr Pro Ser Asp Ile Ala

Val Glu Trp Glu Ser Asn Gly Gln Pro Glu Asn Asn Tyr Lys Thr Thr

Pro Pro Val Leu Asp Ser Asp Gly Ser Phe Phe Leu Tyr Ser Arg Leu

Thr Val Asp Lys Ser Arg Trp Gln Glu Gly Asn Val Phe Ser Cys Ser

Val Met His Glu Ala Leu His Asn His Tyr Thr Gln Lys Ser Leu Ser

Leu Ser Leu Gly Lys

## Claims

1. A liquid pharmaceutical formulation comprising a human growth hormone (hGH) fusion protein, poloxamer 188, and polysorbate 80.

2. The liquid pharmaceutical formulation of claim 1, wherein the hGH fusion protein includes hGH and an immunoglobulin Fc polypeptide.

3. The liquid pharmaceutical formulation of claim 2, wherein the hGH has an amino acid sequence of SEQ ID NO: 2.

4. The liquid pharmaceutical formulation of claim 2, wherein the immunoglobulin Fc polypeptide has an amino acid sequence of SEQ ID NO: 3.

5. The liquid pharmaceutical formulation of claim 1, wherein the hGH fusion protein has an amino acid sequence of SEQ ID NO: 1.

6. The liquid pharmaceutical formulation of claim 1, wherein the formulation includes 30 mg/mL to 150 mg/mL of the hGH fusion protein.

7. The liquid pharmaceutical formulation of claim 1, wherein the formulation includes 0.01% to 0.2% (w/v) poloxamer 188.

8. The liquid pharmaceutical formulation of claim 1, wherein the formulation includes 0.01% to 0.2% (w/v) polysorbate 80.

9. The liquid pharmaceutical formulation of claim 1, wherein the formulation further comprises one or more excipients selected from the group consisting of histidine, arginine, glutamic acid, and sodium chloride (NaCl).

10. The liquid pharmaceutical formulation of claim 1, wherein the formulation further comprises a preservative.

11. The liquid pharmaceutical formulation of claim 10, wherein the preservative is one or more selected from the group consisting of m-cresol, phenol, and benzyl alcohol.

12. The liquid pharmaceutical formulation of claim 11, wherein the preservative is 1 mg/mL to 2 mg/mL of phenol.

13. The liquid pharmaceutical formulation of claim 9, wherein the formulation includes 1 mM to 20 mM histidine.

14. The liquid pharmaceutical formulation of claim 9, wherein the formulation includes 40 mM to 70 mM arginine.

15. The liquid pharmaceutical formulation of claim 9, wherein the formulation includes 40 mM to 70 mM glutamic acid.

16. The liquid pharmaceutical formulation of claim 9, wherein the formulation includes 80 mM to 100 mM sodium chloride.

17. The liquid pharmaceutical formulation of claim 9, wherein the formulation has a pH of 5.5 to 7.0.

18. The liquid pharmaceutical formulation of claim 17, wherein the formulation has a pH of 6.0 to 6.2.

19. The liquid pharmaceutical formulation of claim 1, wherein the formulation forms less than 1.5% of aggregates when stored in refrigerated conditions for 6 months.

20. The liquid pharmaceutical formulation of claim 1, wherein the formulation is administered subcutaneously or intramuscularly.

21. The liquid pharmaceutical formulation of claim 1, wherein the formulation is stored in a container selected from the group consisting of a bottle, a microtube, a bag, a vial, a cartridge, an injector, and a syringe.
